# EUROPÄISCHE PATENTSCHRIFT

(11) **EP 0 353 570 B1**
(45) Veröffentlichungstag und Bekanntmachung des Hinweises auf die Patenterteilung: **14.09.1994**
(21) Anmeldenummer: 89113491.8
(22) Anmeldetag: 22.07.1989
(51) Int. Cl.: G01N 33/545, G01N 33/552, C12N 11/00

(54) **Mit Reagenz ablösbar imprägnierte Trägermatrix**
Support matrix impregnated with releasable reagents
Matrice de support imprégnée de réactifs pouvant être libérés

(30) Priorität: 30.07.1988 DE 3826055
(43) Veröffentlichungstag der Anmeldung: 07.02.1990
(73) Patentinhaber: BOEHRINGER MANNHEIM GMBH, 68298 Mannheim (DE)
(72) Erfinder: Wilk, Hans-Erich, Dr., D-6143 Lorsch (DE); Mangold, Dieter, Dr., D-6701 Maxdorf (DE); Lerch, Rolf, D-6804 Ilvesheim (DE); Steinbiss, Joachim, Dr., D-6143 Lorsch (DE)

(56) Entgegenhaltungen:
- EP-A- 0 239 002
- EP-A- 0 258 683

## Beschreibung

Die Erfindung betrifft eine mit Reagenz ablösbar imprägnierte Trägermatrix, ein Verfahren zu deren Herstellung sowie deren Verwendung.

Speziell ist Gegenstand der Erfindung die Verwendung einer speziellen Trägermatrix für die ablösbare Imprägnierung mit Reagenzien.

In der klinischen Diagnostik sowie auch in der Analyse von Lebensmitteln, Bedarfsgegenständen und Wasser werden viele häufig wiederkehrende Parameter bestimmt. Dazu werden oft Nachweisverfahren unter Verwendung von Enzymen oder Nachweisverfahren unter Verwendung immunologisch aktiver Substanzen durchgeführt. Für diese ständig durchzuführenden Bestimmungen sind schon vorbereitete Testkits im Handel, die alle für eine Analyse erforderlichen Komponenten enthalten. In der einfachsten Form liegen dabei die einzelnen Komponenten in Form von Lösungen vor, die in entsprechenden Mengen mit der zu untersuchenden Probe gemischt werden. Viele Substanzen, insbesondere biologisch aktive Moleküle sind jedoch in Lösung nicht stabil und können in dieser Form nicht über einen längeren Zeitraum aufbewahrt werden. Um diesen Nachteil zu vermeiden, werden solche Substanzen oft in trockener Form gelagert und erst unmittelbar vor Durchführung der Analyse in einer entsprechenden Flüssigkeit gelöst und als Lösung eingesetzt. Zum Beispiel können so Lyophilisate verwendet werden. Nachteil der Lyophilisate ist jedoch das Verfahren zu ihrer Herstellung, das sehr aufwendig und teuer ist.

Bekannt ist es auch, Substanzen in fester Form zu Tabletten zu verpressen und so fertige Dosiereinheiten herzustellen. Probleme ergeben sich aber häufig, wenn diese Tabletten zu hart gepreßt sind, weil sie sich dann nur schwer auflösen. Sind sie andererseits nicht hart genug gepreßt, weisen die Tabletten eine ungenügende Härte auf und bröckeln ab, so daß die Dosierung ungenau wird.

Um diese Nachteile zu vermeiden, wurde bereits vorgeschlagen, Papiervliese mit Reagenzien zu imprägnieren und dann diese Papiervliese in die Reaktionslösung während des Bestimmungsverfahrens einzubringen. Dazu muß das Reagenz einerseits auf dem Papiervlies sehr gut haften, damit nicht durch vorzeitige Ablösung die Menge, die auf dem Vlies imprägniert ist, verändert wird. Andererseits ist es notwendig, daß bei Einbringen in die Reaktionslösung das aufgebrachte Reagenz schnell und vollständig eluiert wird. Die bisher bekannten Papiervliese befriedigen in dieser Hinsicht nicht, da sie entweder das aufgebrachte Reagenz zu wenig binden, so daß schon während der Lagerung ein Teil des aufgebrachten Reagenzes sich ablöst, oder aber die Bindung des Reagenzes ist so stark, daß es nicht schnell und vollständig eluiert werden kann.

In den letzten Jahren wurden vor allem in der klinischen Diagnostik zur Untersuchung von Körperflüssigkeiten, wie Urin, Blut oder von Blut abgeleiteten Proben, wie Serum oder Plasma, zunehmend sogenannte trägergebundene Tests eingeführt. Bei diesen liegen trockene Reagenzien in oder auf mindestens einer festen Testschicht vor, die mit der Probe in Kontakt gebracht wird. Je nach der Aufgabe, die eine solche Testschicht im trägergebundenen Test erfüllen soll, können die Reagenzien auf der als Reagenzträger fungierenden Testschicht in fixierter Form oder eluierbar vorliegen.

Aus EP-A-0 262 445 ist beispielsweise ein mehrschichtiger Testträger für die Analyse von Flüssigkeiten, insbesondere Körperflüssigkeiten, wie Blut und Urin, bekannt, der eine flüssigkeitsabsorbierende Schicht enthält, die die Probenflüssigkeit aufnimmt und in dem die für die Nachweisreaktion erforderlichen Reagenzien in verschiedenen, voneinander getrennten Schichten auf unterschiedlichen Trägern untergebracht sind. Um die Reagenzien ausschließlich in der flüssigkeitsabsorbierenden Schicht mit den nachzuweisenden Bestandteilen der Probe zur Reaktion zu bringen, ist es erforderlich, daß die Reagenzien bei Kontakt mit der zu untersuchenden Flüssigkeit vollständig von der Testschicht in die flüssigkeitsabsorbierende Schicht gelangen. Als geeignete Testschichten werden neben mit reagenzimprägniertem Papier insbesondere solche vorgeschlagen, die die Reagenzien in einen Film aus wasserlöslichem Material eingebettet enthalten. Der Film soll aus hochmolekularem, polymerem Material bestehen. Als bevorzugt wird Xanthan beschrieben. Ein Nachteil solcher Testschichten besteht darin, daß bei dem in Lösung gehen der Reagenzien auch das filmbildende Material in die zu untersuchende Probe gelangt. Dies kann unter Umständen zu Störungen führen, z. B. wenn das filmbildende Material mit zu bestimmenden Probeninhaltsstoffen wechselwirkt. Speziell hochmolekulare, polymere, wasserlösliche Materialien erhöhen die Viskosität der lösenden Flüssigkeit bereits in niedrigen Konzentrationen beträchtlich. Die Nachweisreaktionen laufen diffusionskontrolliert dann oft sehr langsam ab. Die Lösung des Reagenzes aus dem als Träger fungierenden Film durch Diffusion kann erheblich verzögert werden. Oft ist es aber gerade angestrebt, daß die Reagenzien bei Kontakt mit einer Flüssigkeit sehr schnell in Lösung gehen und die Nachweisreaktion sehr schnell erfolgt.

Dies sollte erfindungsgemäß dadurch erreicht werden, daß die Reagenzien von der Oberfläche eines unlöslichen Trägers her abgelöst werden können. Außerdem sollten die Reagenzien auf den entsprechenden Träger ohne aufwendige Verfahren möglichst einfach aufgebracht werden können, wie es z. B. durch Imprägnierung des Trägers mit entsprechenden Lösungen der Reagenzien der Fall ist. Aufgabe der vorliegenden Erfindung war es deshalb, eine solche mit Reagenz ablösbar imprägnierte Trägermatrix, d. h. ein solches festes, unter Einsatzbedingungen wasserunlösliches Material, das das Reagenz nach Imprägnierung auf der Oberfläche trägt, zur Verfügung zu stellen, die lange Zeit gelagert werden kann, ohne daß das imprägnierte Reagenz wesentlich an Aktivität verliert und die eine schnelle und vollständige Ablösung des Reagenzes unter Erhalt dessen Aktivität gestattet.

Insbesondere war es Aufgabe der vorliegenden Erfindung, eine solche mit Reagenz ablösbar imprägnierte Trägermatrix zur Verfügung zu stellen, die in Testvorrichtungen und Verfahren zur Bestimmung von Probeninhaltsstoffen verwendet werden kann.

Speziell bestand die Aufgabe der Erfindung darin, eine solche Trägermatrix zur Verfügung zu stellen, die für die ablösbare Imprägnierung mit Reagenzien geeignet ist.

Überraschenderweise wurde festgestellt, daß eine mit Reagenz imprägnierte Trägermatrix aus Polyvinylalkohol (PVA)-beschichtetem Glas, die gestellte Aufgabe erfüllt, bei dem der PVA nur langsam oder überhaupt nicht unter 20° C in Wasser löslich ist. Eine derartige Trägermatrix führt zu einer solchen Stabilisierung der imprägnierten Reagenzien, daß auch nach langer Lagerung und sogar nach Lagerung bei erhöhter Temperatur kein wesentlicher Aktivitätsverlust auftritt. Außerdem trägt das PVA-beschichtete Glas darauf imprägnierte Reagenzien dergestalt, daß diese bei Kontakt mit einer Flüssigkeit, insbesondere mit der zu untersuchenden Probenflüssigkeit sehr schnell, gegebenenfalls innerhalb weniger Sekunden vollständig abgelöst werden. Nach Ablösung des Reagenzes von der erfindungsgemäßen imprägnierten Trägermatrix besitzt das Reagenz im wesentlichen die gleichen Eigenschaften wie vor der Imprägnierung auf die Matrix.

Um diese vorteilhaften Eigenschaften zu erreichen, muß die Trägermatrix aus zwei Komponenten bestehen. Die erste ist Glas, das prinzipiell in jeder beliebigen Form und Zusammensetzung vorliegen kann.

Die zweite Komponente ist kaltwasserunlöslicher Polyvinylalkohol, mit dem das Glas beschichtet ist. Polyvinylalkohol wird üblicherweise aus Polyvinylacetat durch Verseifung hergestellt, wobei je nach den gewünschten Eigenschaften des Produktes eine vollständige oder teilweise Verseifung durchgeführt wird. Für den erfindungsgemäßen Zusatz ist sowohl ein voll- als auch ein teilverseiftes Produkt verwendbar. Polyvinylalkohole, die im Handel in großer Menge angeboten werden, unterscheiden sich insbesondere durch ihr durchschnittliches Molekulargewicht, welches normalerweise zwischen etwa 10000 und 100000 liegt und in einigen Sonderfällen auch noch wesentlich höhere Werte erreichen kann, sowie durch den Restgehalt an Acetylgruppen. Die niedermolekularen Verbindungen, die ca. 5 bis 15 %, insbesondere etwa 10 % Acetylgruppen enthalten, sind in Wasser am leichtesten löslich, hochmolekulare und/oder stärker acetylhaltige Produkte sind dagegen in Wasser weniger löslich. Von Einfluß auf die Löslichkeit ist ferner die Wechselwirkung der Polyvinylalkoholketten untereinander. Durch eine parallele Lagerung der Polymerketten in bestimmten Bereichen entstehen "kristalline" Bezirke, wobei die Orientierungstendenz umso größer ist, je regelmäßiger die Ketten aufgebaut sind und je geringer der Anteil der Acetylgruppen ist, die einer Orientierung am stärksten entgegenwirken. Bei einem Verseifungsgrad von 97 - 100 mol %, d. h. bei einem Acetylierungsgrad von 3 - 0 mol % nimmt deshalb die "Kristallinität" besonders stark zu, wodurch umgekehrt die Kaltwasserlöslichkeit stark abnimmt.

Die Wasserlöslichkeit kann weiterhin durch Nachbehandlung mit Aldehyden (Acetalisierung) oder andere chemische Umwandlungen der Alkoholgruppen verringert werden. Erfindungsgemäß brauchbar sind solche Polyvinylalkohole mit einer sehr geringen Kaltwasserlöslichkeit. Die Produkte sollen bei 20 °C in Wasser nur langsam oder überhaupt nicht gelöst werden. Bei Temperaturen von 50 - 100 °C, insbesondere bei Temperaturen von über 60 °C ist eine Löslichkeit in Wasser aber nicht nachteilig.

In der erfindungsgemäß mit Reagenz imprägnierten Trägermatrix ist das Glas so mit einer PVA-Schicht bedeckt, daß die gesamte Glasoberfläche belegt ist. Für optimale Lager- und Reagenzablöseigenschaften ist das Glas mit etwa 0,5 bis 20 Gew.-%, vorzugsweise 1 - 10 Gew.-% PVA-beschichtet.

Erfindungsgemäß kann das PVA-beschichtete Glas als Trägermatrix in jeder beliebigen Form vorliegen. Um eine möglichst große Oberfläche bereitzustellen, kann es aber vorteilhaft sein, daß die Trägermatrix in Form von Fasern ausgebildet ist. Eventuell genügt es für viele Zwecke, die Matrix als Faserknäuel einzusetzen, in der die einzelnen Fasern völlig ungeordnet sind. Oft wird es jedoch erwünscht sein, flächige Reagenzträger zu haben, die blatt- oder schichtenförmig ausgebildet sind. Für diesen Fall ist es ganz besonders bevorzugt, wenn die Trägermatrix ein Vlies ist.

Ein mit kaltwasserunlöslichem PVA beschichtetes Glas, insbesondere solches in Faserform und ganz besonders ein mit PVA beschichtetes Glasfaservlies ist sehr gut zur Imprägnierung mit Reagenzien geeignet. Aus EP-A-0 239 002 sind PVA-beschichtete Glasfaservliese, wie sie zuvor beschrieben sind, bereits bekannt. Sie dienen dort jedoch lediglich zum Transport von Serum und Plasma sowie gegebenenfalls zur Abtrennung von Erythrozyten aus Blut. Die Imprägnierung mit Reagenzien wird in der europäischen Anmeldung nicht angesprochen. Es war deshalb nicht zu erwarten, daß durch Imprägnierung von mit kaltwasserunlöslichem PVA beschichtetem Glas mit Reagenzien solche Reagenzträger erhalten werden, die das der Erfindung zugrundeliegende Problem so gut lösen, wie es erfindungsgemäß der Fall ist. Besondere Vorteile sind dann zu erzielen, wenn das auf die erfindungsgemäße Trägermatrix imprägnierte Reagenz ein Protein ist. Sind Proteine auf einer erfindungsgemäßen Trägermatrix imprägniert, werden sie bei Kontakt mit einer Flüssigkeit, insbesondere der zu untersuchenden Probenflüssigkeit in der Regel sehr schnell und vollständig gelöst und hierbei renaturiert, d. h. sie liegen nach dem Ablösevorgang von der Matrix im wesentlichen in der gleichen biologischen Aktivität vor, wie vor der Imprägnierung der Matrix mit einer entsprechenden Proteinlösung.

Ganz besonders bevorzugte erfindungsgemäß auf die Trägermatrix imprägnierte Reagenzien sind solche für enzymatische Bestimmungen, wie Enzyme und solche für immunologische Nachweisreaktionen, wie Antigene, Antikörper oder/und deren Fragmente sowie Konjugate immunologisch aktiver Substanzen mit Markierungssubstanzen, wie beispielsweise Enzymen. Ebenso können auf die erfindungsgemäße Matrix auch sonstige Bindungspartner, wie z. B. Biotin/Avidin bzw. Streptavidin, Protein A/Immunglobulin G oder Concanavalin A/Mannose beziehungsweise Konjugate dieser Substanzen mit Enzymen oder Antikörpern oder Antikörperfragmenten ablösbar imprägniert sein.

Hervorragende Ergebnisse bezüglich Schnelligkeit und Vollständigkeit der Wiederablösung von Reagenzien von der erfindungsgemäßen Matrix sowie bezüglich Lagerstabilität der erfindungsgemäßen Matrix werden mit Enzymen, insbesondere mit β-Galactosidase und mit Konjugaten aus immunologisch aktiven Substanzen und Markierungssubstanzen, insbesondere mit Konjugaten aus IgG-Molekülen mit β-Galactosidase erhalten.

Neben den eigentlichen "aktiven" Bestandteilen des Reagenzes, wie beispielsweise enzymatisch oder/und biologisch aktiven Proteinen kann das Reagenz auch noch weitere Substanzen enthalten. Insbesondere kann das Reagenz auch solche Stoffe enthalten, die zur Erhaltung der Aktivität bzw. Vermeidung der Desaktivierung des Reagenzes in Lösung geeignet sind bzw. benötigt werden. Hierfür sind insbesondere Puffersubstanzen zur Aufrechterhaltung eines bestimmten pH-Wertes in Lösung, Detergenzien, Salze oder bestimmte Schutzsubstanzen, wie Albumin oder Saccharose zu verstehen.

Die Reagenzkonzentration auf der Trägermatrix kann in weiten Grenzen variiert werden, ohne daß dies einen wesentlichen Einfluß auf die Schnelligkeit und Vollständigkeit der Ablösung des Reagenzes hat. Eine natürliche Grenze der Konzentration nach oben wird dort erreicht, wo das Reagenz nicht mehr fest auf der Oberfläche des PVA-beschichteten Glases haftet und sich bereits vor Gebrauch, noch im trockenen Zustand ablöst.

Die Herstellung einer erfindungsgemäßen mit Reagenz imprägnierten Trägermatrix erfolgt grundsätzlich so, daß Glas zunächst mit kaltwasserunlöslichem PVA beschichtet, das beschichtete Glas mit Reagenz imprägniert und die imprägnierte Matrix abschließend gegebenenfalls getrocknet wird. Speziell für den Fall der Herstellung eines erfindungsgemäßen Vlieses kann ein entsprechendes zuvor hergestelltes Glasfaservlies nachträglich mit einer Lösung des Polyvinylalkohols in Wasser oder einem geeigneten organischen Lösungsmittel behandelt und anschließend getrocknet werden. Aufgrund des bevorzugten Löslichkeitsverhaltens und des Schmelzpunktes von PVA sollte eine solche Behandlung des Glasfaservlieses bei Temperaturen über 60 °C, ganz besonders bei 90 - 140 °C durchgeführt werden.

Vorzugsweise wird ein erfindungsgemäß PVA-beschichtetes Glasfaservlies so hergestellt, daß bereits bei der Fertigung des Glasfaservlieses Polyvinylalkohol in fester Form, besonders bevorzugt in Faserform zu den Glasfasern gegeben wird.

PVA-beschichtete Glasfaservliese für erfindungsgemäße Matrices werden ganz besonders bevorzugt so hergestellt, daß man trockene Glasfasern, die einen mittleren Durchmesser von 0,1 - 20 µm und eine Länge von 0,1 - 5 mm aufweisen, in einem sehr großen Überschuß von Wasser suspendiert, dadurch vereinzelt und diese "Bütte" analog den in der Papierherstellung üblichen Verfahren und mit Hilfe der dort üblichen Maschinen zu dünnen Schichten formt und trocknet. Der Bütte zugesetzte Polyvinylalkoholpulver oder -fasern aus kaltwasserunlöslichem PVA verteilen sich bei der Aufschlemmung der Glasfasern gleichmäßig in der Masse und werden beim anschließenden Herstellen des Vlieses soweit gelöst bzw. aufgeschmolzen, daß sie anschließend an die Trocknung des Vlieses einen vollständigen und gleichmäßigen Überzug auf den Glasfasern bilden. Ein solchermaßen beschichtetes Glasfaservlies ist - was die Saugfähigkeit und den Transport von Wasser bzw. wässrigen Lösungen durch dieses Vlies anbelangt - gegenüber einem unbeschichteten Glasfaservlies nicht beeinträchtigt.

Da der Polyvinylalkohol die Glasfasern relativ gleichmäßig überzieht, wenn er wie vorstehend aufgebracht wird, reichen bereits kleine Mengen, insbesondere etwa 0,5 - 20 Gew.-%, vorzugsweise 2,5 - 10 Gew.-% aus, um die Fasern vollständig mit einem PVA-Mantel zu umgeben. Anteile von über 20 Gew.-% wären zwar für den beabsichtigten Effekt nicht schädlich, sind aber aus verarbeitungstechnischen Gründen oft nicht wünschenswert, da z. B. Glasfaservliese mit einem hohen PVA-Gehalt sehr starr sind.

Zur Imprägnierung der Trägermatrix mit Reagenz wird diese vorzugsweise mit einer Lösung des Reagenzes getränkt, wobei z. B. die Tränklösung auf die Trägermatrix aufgegeben oder diese in die Tränklösung eingetaucht wird. Um eine möglichst homogene Imprägnierung der Trägermatrix zu erreichen, wird der letzteren Ausführungsform der Vorzug gegeben.

Als Lösungsmittel kann jede Flüssigkeit gewählt werden, die das Reagenz ausreichend gut löst, die die Eigenschaften des Reagenzes nicht negativ beeinflußt und die nach Imprägnierung der Trägermatrix mit dem Reagenz wieder so entfernt werden kann, daß Wiederablöseeigenschaften und Aktivität des Reagenzes nicht beeinträchtigt werden. Im Falle enzymatisch oder/und immunologisch aktiver Substanzen ist das Lösungsmittel der Wahl Wasser.

Nach dem Imprägniervorgang schließt sich eine Trocknungsstufe an, wenn das Lösungsmittel des Reagenzes gesondert entfernt werden muß. Im Falle von Wasser als Lösungsmittel ist dies regelmäßig der Fall. Es muß hierbei je nach Art und Zusammensetzung des Reagenzes entschieden werden, bei welchen Temperaturen der Trocknungsvorgang durchgeführt werden und wie lange er dauern soll. Insbesondere bei Reagenzien mit enzymatisch oder/und immunologisch aktiven Substanzen sollten die Temperaturen etwa 70 °C und die Trocknungsdauer etwa 1 Stunde nicht übersteigen.

Die erfindungsgemäße mit Reagenz imprägnierte Trägermatrix kann überall dort verwendet werden, wo in einer Flüssigkeit ein Inhaltsstoff mit Reagenzien zur Umsetzung gebracht werden soll. Insbesondere und besonders vorteilhaft ist sie dort einsetzbar, wo Reagenzien einer Flüssigkeit in leicht handbarer Weise, vordosiert und durch Lagerung in der Aktivität nicht beeinträchtigt, zugegeben werden sollen. Ganz besondere Vorteile bietet die Verwendung der erfindungsgemäßen mit Reagenz ablösbar imprägnierten Trägermatrix in Testvorrichtungen und Verfahren zur Bestimmung von Probeninhaltsstoffen, sofern diese in einer Flüssigkeit vorliegen. Hervorragend geeignet ist die erfindungsgemäße Matrix insbesondere zur Verwendung als Reagenzträger in trägergebundenen Tests zur enzymatischen oder/und immunologischen Bestimmung von Probeninhaltsstoffen.

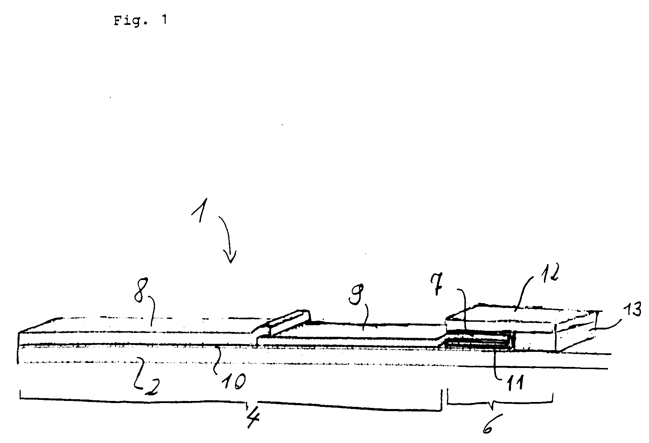

Die Erfindung eignet sich vor allem für die Verwendung in einem Testträger zur Durchführung immunologischer Bestimmungen, da es dort sehr wesentlich auf die schnelle und vollständige Eluierbarkeit von Reagenzien insbesondere immunologisch oder/und enzymatisch aktiver Substanzen, wie z. B. Antikörper-Enzym-Konjugaten von Trägermatrices ankommt. Ein solcher Testträger wird im folgenden anhand eines in Figur 1 schematisch dargestellten Ausführungsbeispiels näher erläutert. Es zeigt Fig. 1 eine perspektivische Darstellung eines Testträgers zur Durchführung immunologischer Bestimmungen

Der dargestellte Testträger 1 hat eine Basisschicht 2, auf der die übrigen Testschichten befestigt sind. In seiner Längsrichtung läßt sich der Testträger in einen Probenaufgabebereich 4 und in einen Auswertebereich 6 unterteilen. In dem Probenaufgabebereich 4 sind nebeneinander eine Konjugatschicht 8 und eine Flüssigkeitstransportschicht 9 auf der Basisschicht 2 mit Hilfe von Schmelzkleber 10 befestigt. Die Schicht 8 überlappt gerinfügig die anschließende Schicht 9 um einen möglichst guten Fluidkontakt zwischen ihnen zu gewährleisten. Die Schichten 8 und 9 bilden eine Flüssigkeitstransportstrecke, die sich von dem Probenaufgabe- und Vorreaktionsbereich 4 in den Auswertebereich 6 erstreckt.

Im dargestellten Beispielsfall wird die Probe auf die Konjugatschicht 8 aufgegeben, wobei diese Schicht gleichzeitig dazu dient, eine erste Reaktionsstufe durchzuführen. Der Probenaufgabebereich 4 dient zugleich als Vorreaktionsbereich.

Im den Auswertebereich 6 sind auf der Basischicht 2 übereinander eine Farbbildungsschicht 11, eine Abdeckschicht 7 und eine Niederhalterschicht 12 zu erkennen. Die Niederhalterschicht 12 besteht aus einer verhältnismäßig steifen Kunststoffolie. Sie ist mit Hilfe eines Schmelzkleberstreifens 13 entsprechend großer Schichtdicke an der Basisschicht 2 so befestigt, daß sie parallel zu ihr mit einem Abstand verläuft, der etwa der Gesamtdicke von Farbbildungsschicht 11 und Abdeckschicht 7 entspricht. Die Niederhalterschicht 12 hat eine ausreichende Steifigkeit, um die zwischen ihr und der Basisschicht 2 befindlichen Schichten so zusammenzudrücken, daß ein guter Fluidkontakt zwischen ihnen gewährleistet ist.

Bei der dargestellten bevorzugten Ausführungsform sind neben der Farbbildungsschicht auf ihrer in Längsrichtung der Basisschicht 2 von dem Probenaufgabebereich 4 abgewandten Seite (also in Fig. 1 rechts von der Farbbildungsschicht 11) keine weiteren saugfähigen Schichten vorgesehen. Die Farbbildungsschicht 11 steht also mit dem in Flüssigkeitstransportrichtung letzten Teilstück der Flüssigkeitstransportstrecke 8,9,7 in Fluidkontakt.

Die Farbbildungsschicht 11 besteht in der dargestellten bevorzugten Ausführungsform aus einer Tragfolie und einer darauf befindlichen verzögert löslichen Filmschicht, die ein Farbbildungsreagenz enthält.

Der in der Figur dargestellte Testträger ist insbesondere für immunologische Bestimmungen geeignet. Derartige Bestimmungen nutzen hochspezifische Bindungsreaktionen zwischen verschiedenen Spezies aus, die man als Bindungspartner bezeichnen kann. Immunologische Bindungspartner sind insbesondere Antikörper einerseits sowie Antigene oder Haptene andererseits.

Für den Fall, daß ein in der Probe enthaltenes Antigen AG als Analyt bestimmt werden soll, ist beispielsweise folgender Testablauf typisch.

Die Probe wird auf die Konjugatschicht 8 aufgegeben. Die Konjugatschicht enthält ein lösliches Konjugat AKE eines mit dem AG spezifisch bindungsfähigen Antikörpers AK mit einem Enzym E. Durch die spezifische Bindungsreaktion entstehen Komplexe AG-AKE.

Überschüssiges AKE gelangt zusammen mit den AG-AKE-Komplexen in die Flüssigkeitstransportschicht 9. Diese enthält ein Antigen AGF in trägerfixierter Form. Das AGF ist mit dem Probenantigen identisch oder analog zu diesem, d. h. mit dem Antikörper des in der Konjugatschicht 8 enthaltenen AKE spezifisch bindungsfähig.

Das überschüssige freie AKE wird nun aufgrund der spezifischen Bindungsreaktion mit dem der Schicht 9 fixierten Antigen trägerfixiert. Für die Funktion ist es wichtig, daß die Belegungsdichte des fixierten Antigens auf der Schicht 9 hoch genug ist, daß praktisch das gesamte überschüssige Konjugat daran gebunden wird. Die Schicht 9 kann deshalb auch als "Fixierungsschicht" bezeichnet werden. Nur die freien AG-AKE-Komplexe gelangen in die Auswertezone 6. Dadurch entspricht die Menge der in der Auswertezone 6 eintreffenden AG-AKE-Komplexe (und damit die Menge des Markierungsenzyms) der Menge des Analyten.

Die Probenflüssigkeit mit den AG-AKE-Komplexen strömt weiter in die Abdeckschicht 7 und füllt diese vollständig, im wesentlichen bevor die Farbbildungsreaktion mit dem Farbbildungsreagenz in der Schicht 11 beginnt. Der verzögerte Beginn der Farbbildungsreaktion wird, wie oben dargelegt, insbesondere dadurch erreicht, daß sich die Schicht 11 verzögert löst.

Die Abdeckschicht 7 ist mit der Fixierungsschicht 9 einstückig hergestellt, d. h. beide Schichten bestehen aus einem Streifen des gleichen Schichtmaterials. Dies ist bevorzugt, jedoch nicht notwendig. Die Abdeckschicht 7 könnte auch eine separate Schicht sein, die in irgendeiner Weise mit der Flüssigkeitstransportstrecke 8,9 in Fluidkontakt steht.

Die Flüssigkeit dringt senkrecht zu den Schichtoberflächen in die Farbbildungsschicht 11 ein. Die Farbbildungsschicht 11 enthält ein Substrat für das Enzym E. In Abhängigkeit von der Enzymkonzentration findet ein Farbumschlag statt, der ein Maß für die Konzentration des Analyten ist.

Die visuelle oder apparative Auswertung des Farbumschlags kann von der Seite der Basisschicht oder von der Abdeckschicht her erfolgen. Je nach Ausführungsart muß hierfür die Basis- oder die Abdeck- und Niederhalterschicht so beschaffen sein, daß man durch sie die Farbänderung in der Farbbildungsschicht feststellen kann.

Als Konjugatschicht 8 hat sich die erfindungsgemäße imprägnierte Trägermatrix sehr bewährt. Insbesondere in seiner Ausführungsform als ein mit kaltwasserunlöslichem PVA beschichtetes Glasfaservlies, das mit einem Antikörper-Enzym-Konjugat imprägniert ist, gewährleistet es die schnelle und vollständige Eluierbarkeit des AKE. Dies ist sehr wesentlich, da die Probenflüssigkeit in nur wenigen Sekunden durch die Konjugatschicht 8 in die Schicht 9 gesaugt wird und es für die vorstehend beschriebene immunologische Bestimmungsmethode darauf ankommt, daß das Antikörper-Enzym-Konjugat vollständig eluiert wird.

Die Funktionsweise des Testträgers wurde vorstehend beispielhaft für den Fall beschrieben, daß ein Antigen bestimmt werden soll. Ein analoger Testverlauf ist auch zur Bestimmung eines Antikörpers möglich, wobei dann ein Antigenkonjugat in der Schicht 8 und ein trägerfixierter analoger Antikörper in der Schicht 9 eingesetzt werden müßte.

Der beschriebene immunologische Testablauf ist - abgesehen von den Besonderheiten der jeweiligen Erfindung - ähnlich dem in der DE-A 36 38 654 beschrieben.

Die vorstehend beschriebene Testvorrichtung ist als Nachweiseinheit für einen Testkit zur Bestimmung eines Analyten im Stuhl besonders geeignet, wie er in der deutschen Patentanmeldung P 37 16 891 beschrieben wird. Dieser Testkit hat eine Probensammeleinheit, in der aus den Stuhlproben durch Elution mit Hilfe eines Elutionsmittels eine Flüssigkeit gewonnen wird, die den Analyten enthält. Die so gewonnene Probenflüssigkeit kann vorteilhaft mit einer Testvorrichtung untersucht werden, wie sie zuvor beschrieben wurde.

Die Erfindung wird durch die folgenden Beispiele erläutert:

### Beispiel 1

### Herstellung der Trägermatrix

1 kg Glasfasern Type 108 E (John Mansville, Denver/USA), 0,05 kg Polyvinylalkoholfasern Typ Kuralon VPB 105-2 (Rohtext Textil GmbH, Mönchengladbach, BRD) werden in 1000 l destilliertem Wasser suspendiert. Zur Herstellung des Vlieses wird eine Schrägsiebmaschine (VOID, Heidenheim, BRD) verwendet. Die Suspension wird zur Blattbildung auf ein Schrägsieb gepumpt. Während die Flüssigkeit abfließt bzw. durch Vakuum abgesaugt wird, orientieren sich die Fasern auf der Sieboberflache und werden als Vlies über Trockenzylinder getrocknet. Die Trocknung findet bei 125 °C statt, bis eine Endfeuchte von 0,5 bis 1,5 Gew.-% erreicht ist. Dabei schmilzt das Kuralon und legt sich als Film auf die Glasoberfläche. Die Absaug- und Transportgeschwindigkeit werden so gewählt, daß ein Material eines Flächengewichtes von 30 g/m² und einer Dicke von 0,25 mm entsteht.

### Beispiel 2

### Stabilität imprägnierten Reagenzes

Es wurden mit Reagenz imprägnierte Träger aus Papier (Type 4210, Firma Kalff, Bundesrepublik Deutschland) multifilem Polyestergewebe (PE 14/100, Schweizer Seidengazefabrik, Thal, Schweiz) und aus PVA-beschichtetem Glas aus Beispiel 1 hergestellt. 6 x 6 mm große Stücke dieser Materialien wurden hierzu mit jeweils 10 µl einer Lösung getränkt, die folgende Bestandteile enthielt:
10 mmol/l HEPES, 25 mmol/l Natriumchlorid, 1 mmol/l Magnesiumaspartat, 2 % Saccharose, 0,5 % Crotein C und β-Galactosidase bei einem pH-Wert der gesamten Lösung von 7,25.

Sofort nach der Tränkung wurden die Vliese 30 Minuten bei 35 °C im Umluft-Trockenschrank getrocknet und nach Abkühlung auf Raumtemperatur sowie nach verschiedenen Belastungen untersucht.

Die β-Galactosidase-Aktivität wurde nach Totalelution (dreimaliges Waschen mit je 50 µl des vorstehend genannten Puffers) im Zentrifugalphotometer mit 50 µl des Eluats nach Zugabe von 5 mmol/l Chlorphenolrot-β-D-galactosid (hergestellt nach EP-A-0 146 866) bestimmt. Es wurden die in Tabelle 1 wiedergegebenen Meßwerte erhalten:

**Tabelle 1**

| | β-Galactosidase-Aktivität in Milliextinktionseinheiten (mE) | | |
|---|---|---|---|
| | unmittelbar nach Tränkung und Trocknung | nach 1 Woche bei 45 °C | nach 3 Wochen bei 45 °C |
| Papiervlies | 1970 | 1700 | 1510 |
| PE 14/100 | 680 | 618 | 512 |
| PVA-beschichtetes Glasfaservlies | 1231 | 1156 | 1216 |

Während für die erfindungsgemäße Trägermatrix auch nach 3 Wochen bei 45 °C praktisch kein Aktivitätsverlust feststellbar ist, nimmt die Enzymaktivität bei imprägniertem Papiervlies und bei imprägniertem Polyestergewebe um über 20 % ab.

Bei Einsatz von Antikörper-Enzymkonjugaten auf PVA-beschichteten Glasfaservliesen bleibt auch die immunologische Aktivität unverändert.

### Beispiel 1

### Eluierbarkeit des Reagenzes von Reagenzimprägnierten Trägermatrices

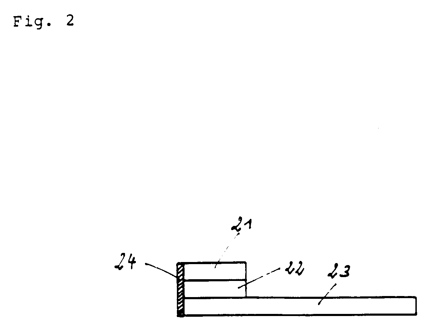

Die Eluierbarkeit eines Reagenzes von entsprechend imprägnierten Reagenzträgern wurde durch Aufgeben einer Serumprobe auf eine Testvorrichtung gemäß Figur 2 gemessen.

In Figur 2 bedeutet 21 eine Aufgabezone, 22 eine reagenzimprägnierte Trägermatrix und 23 ein Saugvlies. 21, 22 und 23 werden durch einen Schmelzkleberstreifen 24 zusammengehalten.

Die Aufgabezone besteht aus einem 6 x 6 mm Glasfaservlies (Type 108, Firma Binzer, Bundesrepublik Deutschland) mit einem Flächengewicht von 60 g/m². Das Saugvlies (17 x 6 mm) besteht aus dem gleichen Material wie die Aufgabezone, besitzt aber ein Flächengewicht von 30 g/m². 22 (6 x 6 mm) besteht
im Fall a) aus einer gemäß Beispiel 1 hergestellten Trägermatrix,
im Fall b) aus einem Papier (Type 4210, Firma Kalff, Bundesrepublik Deutschland)
im Fall c) aus einem Nylongewebe (Nylon 20 HC, Schweizer Seidengazefabrik, Thal, Schweiz) und
im Fall d) aus einem Polyestergewebe (PE2F777, Schweizer Seidengazefabrik, Thal, Schweiz)
die jeweils mit einer Lösung aus 10 mmol/l HEPES, 25 mmol/l Natriumchlorid, 1 mmol/l Magnesiumaspartat, 2 % Saccharose, 0,5 % Crotein C und einem Konjugat aus polyklonalen Schaf-anti-Humanserumalbumin-Antikörpern mit β-D-Galactosidase (IgG<hSA>-β-D-Galactosidase) bei pH 7,25 imprägniert worden sind. Die Dicke der Schichten 21, 22 und 23 beträgt jeweils etwa 0,25 mm.

Zur Messung der Eluierbarkeit des in 22 imprägnierten Reagenzes wurden 64 µl Serum (PNU, Boehringer Mannheim GmbH, Mannheim, Bundesrepublik Deutschland) auf 21 aufgebracht. In jedem der Fälle a) - d) war das Saugvlies bereits nach etwa 25 Sekunden mit Flüssigkeit gefüllt. Zu diesem Zeitpunkt wurde 23 mittels einer Pinzette von der Testvorrichtung abgetrennt und auszentrifugiert (Eppendorff-Laborzentrifuge, 30 Sekunden bei 10000 Umdrehungen pro Minute). Die Enzymaktivität in dem so erhaltenen Eluat wurde nach Zugabe von 5 mmol/l Chlorphenolrot-β-D-galactosid (hergestellt nach EP-A-0 146 866) photometrisch bestimmt.

Es wurden die in Tabelle 2 wiedergegebenen prozentualen Anteile der ursprünglich auf die Trägermatrix imprägnierten Enzymaktivität wiedergefunden.

**Tabelle 2**

| | wiedergefundene Enzymaktivität in % |
|---|---|
| PVA-beschichtetes Glasfaservlies | 106 |
| Papier | 47 |
| Nylongewebe | 83 |
| Polyestergewebe | 80 |

Das auf PVA-beschichtetes Glasfaservlies imprägnierte Reagenz zeigt nicht nur mit Abstand die beste Wiederfindungsrate. Das Reagenz wird darüber hinaus auch quantitativ eluiert.

### Beispiel 4

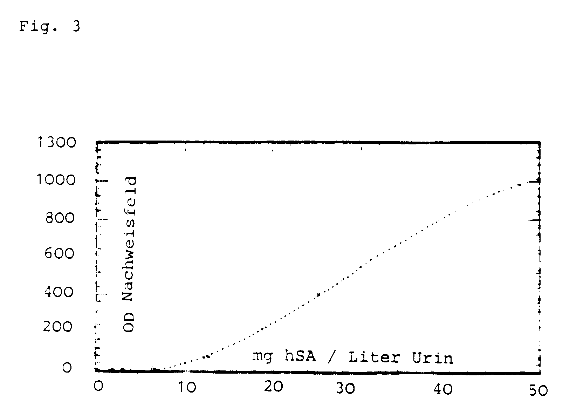

Ein Testträger gemäß Figur 1 wird wie folgt hergestellt:
a) Konjugatschicht 8:
   Ein PVA-beschichtetes Glasfaservlies gemäß Beispiel 1 wird mit einer Lösung von IgG<hSA>-β-D-Galactosidase-Konjugat in 10 mmol/l HEPES, 25 mmol/l Natriumchlorid, 1 mmol/l Magnesiumaspartat, 2 % Saccharose, 0,5 % Crotein C, pH 7,25 getränkt und getrocknet.
   Die Testschichtgröße auf dem Testträger beträgt 20 x 6 mm. Die Konjugatschicht enthält 200 mU β-D-Galactosidaseaktivität.
b) Fixierungsschicht 9 und Abdeckschicht 7:
   An eine Membran aus hydrophilem Polyvinylidendifluorid (PVDF) der Firma Millipore (Bedford), USA) die unter dem Markennamen Immobilon AV vertrieben wird, wird hSA kovalent fixiert. Die Flächenkonzentration wird über die Konzentration in dem für den Tränkungsvorgang verwendeten Puffer auf 20 µg hSA/cm² eingestellt. Die Schichtgröße beträgt 20 x 6 mm.
c) Signalbildungsschicht 11:
   Eine filmbildende Beschichtungsmasse wird hergestellt auf Basis von 0,6 % Ketrol F der Firma Kelco, Hamburg, Bundesrepublik Deutschland (BRD) unter Zusatz von 2,5 % Methylcellulose 15 der Firma Serva, Heidelberg, BRD. Sie enthält 12 mM ChlorPhenolRot-β-Galaktosid (CPRG) und ist in HEPES gepuffert. Die Beschichtungsmasse wird in einer Filmschichtstärke von 200 µm auf eine 100 µm starke Tragfolie aus Pokalon der Firma Lonza, Weil/Rhein, BRD, geschichtet. Die Schichtgröße beträgt 6 x 6 mm.
d) Niederhalterschicht 12:
   Diese besteht aus einer 140 µm starken Pokalonfolie.
   Als Basisschicht wird eine Polyesterfolie "Melinex" der Firma ICI, Frankfurt, BRD, verwendet. Die Verklebung der Komponenten erfolgt mit Schmelzkleber Dynapol S 1358 der Firma Dynamid Nobel, Troisdorf, BRD.
   Bei Aufgabe einer flüssigen Humanserumalbumin (hSA) enthaltenden Probe auf die Konjugatschicht 8 kann in der Signalbildungsschicht 11 nach wenigen Minuten eine Farbänderung von gelb nach rot beobachtet werden.
   Durch Vermessung von Proben bekannten hSA-Gehalts konnte die in Figur 3 dargestellte Eichkurve ermittelt werden.

## Patentansprüche

1. Mit Reagenz ablösbar imprägnierte Trägermatrix, dadurch gekennzeichnet, daß die Trägermatrix aus Glas besteht, das mit Polyvinylalkohol (PVA) beschichtet ist, welcher unter 20° C in Wasser nur langsam oder überhaupt nicht löslich ist.

2. Mit Reagenz ablösbar imprägnierte Trägermatrix gemäß Anspruch 1, dadurch gekennzeichnet, daß das Glas in Faserform vorliegt.

3. Mit Reagenz ablösbar imprägnierte Trägermatrix gemäß Anspruch 2, dadurch gekennzeichnet, daß die Glasfasern als Vlies verarbeitet sind.

4. Mit Reagenz ablösbar imprägnierte Trägermatrix gemäß einem der Ansprüche 1 - 3, dadurch gekennzeichnet, daß das Glas mit 0,5 bis 20 Gew.-% vorzugsweise 2,5 - 10 Gew.-% PVA-beschichtet ist.

5. Mit Reagenz ablösbar imprägnierte Trägermatrix gemäß einem der Ansprüche 1 - 4, dadurch gekennzeichnet, daß das Reagenz ein Protein enthält.

6. Mit Reagenz ablösbar imprägnierte Trägermatrix gemäß einem der Ansprüche 1 - 5, dadurch gekennzeichnet, daß das Reagenz β-Galactosidase oder Konjugate von Antikörpern mit β-Galactosidase enthält.

7. Verfahren zur Herstellung einer mit Reagenz ablösbar imprägnierten Trägermatrix gemäß Anspruch 1, dadurch gekennzeichnet, daß Glas mit PVA beschichtet, das beschichtete Glas mit Reagenz imprägniert und gegebenenfalls getrocknet wird.

8. Verfahren zur Herstellung einer mit Reagenz ablösbar imprägnierten Trägermatrix gemäß einem der Ansprüche 2 und 3, dadurch gekennzeichnet, daß Glasfasern in einem Überschuß von Wasser unter Zusatz von Polyvinylalkohol aufgeschwemmt, hiermit nach in der Papierherstellung üblichen Verfahren eine Schicht geformt und unter erhöhter Temperatur getrocknet, sowie die Schicht mit einer Reagenzlösung imprägniert und gegebenenfalls getrocknet wird.

9. Verwendung von PVA-beschichtetem Glas, das mit solchem PVA beschichtet ist, der unter 20° C in Wasser nur langsam oder überhaupt nicht löslich ist, als Träger für ablösbar imprägnierte Reagenzien.

10. Verwendung von PVA-beschichtetem Glas gemäß Anspruch 9, dadurch gekennzeichnet, daß als Reagenzien enzymatisch oder/und immunologisch aktive Substanzen eingesetzt werden.

11. Verwendung einer mit Reagenz ablösbar imprägnierten Trägermatrix gemäß einem der Ansprüche 1 - 6 in Testvorrichtungen und Verfahren zur Bestimmung von Probeninhaltsstoffen.

## Claims

1. Carrier matrix dissolvably impregnated with reagent, characterised in that the carrier matrix consists of glass which is coated with polyvinyl alcohol (PVA) which, below 20°C, is only slowly soluble or not soluble at all in water.

2. Carrier matrix dissolvably impregnated with reagent according to claim 1, characterised in that the glass is present in fibre form.

3. Carrier matrix dissolvably impregnated with reagent according to claim 2, characterised in that the glass fibres are worked up as fleece.

4. Carrier matrix dissolvably impregnated with reagent according to one of claims 1 - 3, characterised in that the glass is coated with 0.5 to 20 wt.%, preferably 2.5 - 10 wt.% of PVA.

5. Carrier matrix dissolvably impregnated with reagent according to one of claims 1 - 4, characterised in that the reagent contains a protein.

6. Carrier matrix dissolvably impregnated with reagent according to one of claims 1 - 5, characterised in that the reagent contains β-galactosidase or conjugate of antibody with β-galactosidase.

7. Process for the production of a carrier matrix dissolvably impregnated with reagent according to claim 1, characterised in that glass is coated with PVA, the coated glass is impregnated with reagent and possibly dried.

8. Process for the production of a carrier matrix dissolvably impregnated with reagent according to one of claims 2 and 3, characterised in that glass fibres are slurried in an excess of water with addition of polyvinyl alcohol, a layer is formed herewith according to processes usual in paper manufacture and dried at elevated temperature, as well as the layer is impregnated with a reagent solution and possibly dried.

9. Use of PVA-coated glass which is coated with such PVA which, below 20°C, is only slowly soluble or not soluble at all in water as carrier for dissolvably impregnated reagents.

10. Use of PVA-coated glass according to claim 9, characterised in that enzymatically and/or immuno-logically active substances are used as reagents.

11. Use of a carrier matrix dissolvably impregnated with reagent according to one of claims 1 - 6 in test devices and processes for the determination of sample components.

## Revendications

1. Matrice de support imprégnée de réactifs pouvant être libérés, caractérisée en ce que la matrice de support est en verre revêtu de poly(alcool vinylique) (PVA) faiblement soluble ou insoluble dans l'eau à une température inférieure à 20°C.

2. Matrice de support imprégnée de réactifs pouvant être libérés, selon la revendication 1, caractérisée en ce que le verre se trouve sous forme de fibres de verre.

3. Matrice de support imprégnée de réactifs pouvant être libérés, selon la revendication 2, caractérisée en ce que les fibres de verre sont transformées en non-tissé.

4. Matrice de support imprégnée de réactifs pouvant être libérés, selon l'une quelconque des revendications 1-3, caractérisée en ce que le verre est revêtu de 0,5 à 20 % en poids, de préférence de 2,5-10 % en poids de PVA.

5. Matrice de support imprégnée de réactifs pouvant être libérés, selon l'une quelconque des revendications 1-4, caractérisée en ce que le réactif contient une protéine.

6. Matrice de support imprégnée de réactifs pouvant être libérés, selon l'une quelconque des revendications 1-5, caractérisée en ce que le réactif contient de la β-galactosidase ou un conjugué d'anticorps et de β-galactosidase.

7. Procédé de préparation d'une matrice de support imprégnée de réactifs pouvant être libérés, selon la revendication 1, caractérisé en ce que le verre est revêtu de PVA, le verre revêtu est imprégné de réactifs et éventuellement séché.

8. Procédé de préparation d'une matrice de support imprégnée de réactifs pouvant être libérés, selon l'une quelconque des revendications 2 et 3, caractérisé en ce que l'on met en suspension des fibres de verre dans de l'eau en excès, en ajoutant du poly(alcool vinylique), formant ainsi une couche selon un procédé usuel dans la fabrication du papier, et on séche cette couche à température élevée, on l'imprègne avec une solution de réactifs et éventuellement on la séche.

9. Utilisation de verre revêtu de PVA, qui est revêtu d'un PVA faiblement soluble ou insoluble dans l'eau à une température inférieure à 20°C, comme support pour des réactifs qui y sont imprégnés et qui peuvent en être libérés.

10. Utilisation de verre revêtu de PVA selon la revendication 9, caractérisée en ce que comme réactifs on utilise des substances ayant une activité enzymatique et/ou immunologique.

11. Utilisation d'une matrice de support imprégnée de réactifs pouvant être libérés, selon l'une quelconque des revendications 1-6, dans des dispositifs de test et des procédés pour la détermination de substances contenues dans un échantillon.
